# FASCICULE DE BREVET EUROPEEN

(11) **EP 1 313 873 B1**
(45) Date de publication et mention de la délivrance du brevet: **26.03.2008**
(21) Numéro de dépôt: 01956637.1
(22) Date de dépôt: 24.07.2001
(51) Int. Cl.: C12N 1/16, C12N 15/53, C12N 9/02, C12P 33/06

(54) **LEVURES MODIFIEES ET UTILISATIONS, NOTAMMENT POUR LA PRODUCTION DE DERIVES STEROIDIENS**
MODIFIZIERTE ZELLEN UND IHRE VERWENDUNGEN, INSBESONDERE ZUR HERSTELLUNG VON STEROIDDERIVATE
MODIFIED YEASTS AND USES THEREOF, IN PARTICULAR FOR PRODUCING STEROID DERIVATIVES

(30) Priorité: 08.08.2000 FR 0010437
(43) Date de publication de la demande: 28.05.2003
(73) Titulaire: Aventis Pharma S.A., 92160 Antony (FR)
(72) Inventeur: DUMAS, Bruno, F-78117 Chateaufort (FR); CAUET, Gilles, F-67370 Grisheim/Souffel (FR); ACHSTETTER, Tilman, 28213 Bremen (DE); DEGRYSE, Eric, F-91560 Crosne (FR)
(74) Mandataire: Bouvet, Philippe
(86) Numéro de dépôt international: PCT/FR2001/002417
(87) Numéro de publication internationale: WO 2002/012536

(56) Documents cités:
- WO-A-99/25865
- DATABASE WPI Section Ch, Week 199011 Derwent Publications Ltd., London, GB; Class B04, AN 1990-079090 XP002165509 & JP 02 031680 A (AGENCY OF IND SCI & TECHNOLOGY), 1 février 1990 (1990-02-01)
- DATABASE WPI Section Ch, Week 199046 Derwent Publications Ltd., London, GB; Class B01, AN 1990-345001 XP002165510 & JP 02 249488 A (SUMITOMO CHEM IND KK), 5 octobre 1990 (1990-10-05)
- WU D-A ET AL: "EXPRESSION AND FUNCTIONAL STUDY OF WILD-TYPE AND MUTANT HUMAN CYTOCHROME P450C21 IN SACCHAROMYCES-CEREVISIAE" DNA AND CELL BIOLOGY, vol. 10, no. 3, 1991, pages 201-210, XP000992872 ISSN: 1044-5498 cité dans la demande
- MAGDOLEN V ET AL: "TRANSCRIPTIONAL CONTROL BY GALACTOSE OF A YEAST GENE ENCODING A PROTEIN HOMOLOGOUS TO MAMMALIAN ALDO-KETO REDUCTASES" GENE (AMSTERDAM), vol. 90, no. 1, 1990, pages 105-114, XP002182803 ISSN: 0378-1119 cité dans la demande
- MIOSGA T ET AL: "A new yeast gene with homology to the aldo-keto reductase protein family" EMBL SEQUENCE DATABA, 4 décembre 1995 (1995-12-04), XP002165506 cité dans la demande
- SAKAKI T ET AL: "EXPRESSION OF BOVINE CYTOCHROME P450C21 AND ITS FUSE ENZYMES WITH YEAST NADPH-CYTOCHROME P450 REDYCTASE IN SACCHAROMYCES CEREVISIAE" DNA AND CELL BIOLOGY, NEW YORK, NY, US, vol. 9, no. 8, 1990, pages 603-614, XP000992939 ISSN: 1044-5498
- SAKAKI T ET AL: "PROGESTERONE METABOLISM IN RECOMBINANT YEAST SIMULTANEOUSLY EXPRESSING BOVINE CYTOCHROMES P450C17 (CYP17A1) AND P450C21 (CYP21B1) AND YEAST NADPH-P450 OXIDOREDUCTASE" PHARMACOGENETICS, CHAPMAN & HALL, LONDON, GB, vol. 1, no. 2, novembre 1991 (1991-11), pages 86-93, XP000992917 ISSN: 0960-314X
- OECHSNER U ET AL: "A nuclear yeast gene (GCY) encodes a polypeptide with high homology to a vertebrate eye lens protein" FEBS LETTERS, vol. 238, no. 1, 26 septembre 1988 (1988-09-26), pages 123-128, XP000647862

## Description

La présente invention concerne le domaine de la biologie et de la pharmacie. Elle concerne notamment de nouvelles compositions et méthodes utiles pour la production de composés stéroïdiens, ou pour la transformation (sélective) de composés stéroïdiens. Elle concerne plus particulièrement de nouvelles souches de levure, des procédés et constructions génétiques pour leur préparation, ainsi que leur utilisation pour la synthèse ou la modification de composés stéroïdiens. Les souches de levure de l'invention permettent d'améliorer l'efficacité de la synthèse ou d'augmenter la sélectivité ou les rendements du procédé, ainsi que la qualité du produit final. Les souches, procédés et composés de l'invention sont utiles dans la recherche, le développement et la production de produits à activité thérapeutique ou prophylactique, chez l'homme ou l'animal, notamment de dérivés stéroïdiens.

La capacité naturelle de microorganismes à transformer les stéroïdes a été largement décrite dans la littérature. A cet égard, ils représentent une alternative avantageuse pour la production de dérivés stéroidiens difficiles à obtenir par synthèse chimique. Les levures sont par ailleurs particulièrement adaptées pour l'expression d'ADNc codant pour des enzymes actives dans les organelles. De ce fait, les levures, telles S. cerevisiae, ont été largement utilisées pour exprimer des ADNc codant des enzymes stéroïdogènes, telles que les P450 microsomaux ou mitochondriaux. De plus, certaines études destinées à exprimer des enzymes impliquées dans la voie de biosynthèse de l'hydrocortisone ont permis de montrer que les levures étaient capables de transformer efficacement certains intermédiaires. Ainsi, l'utilisation de levures transformées permettant l'expression d'une ou plusieurs enzymes de mammifères impliquées dans la voie de biosynthèse des stéroïdes a été décrite par exemple dans la demande EP340878, le brevet US5,137,822 ou dans Dumas et al. En particuler, l'utilisation d'une levure Saccharomyces cerevisiae exprimant le cytochrome P450 C21 adrenal bovin pour produire du 11-deoxycortisol à partir de 17α-hydroxyprogestérone a été précédemment décrite (Database WPI Section Ch., Week 199011 Derwent Publications Ltd., London, GB). De même, les demandeurs ont trouvé que les hydroxystéroides Δ5-3ß tels la prégnénolone, la 17α-hydroxyprégnénolone et la DHEA étaient converties par les levures en esters acétate correspondants. Les demandeurs ont également mis en évidence que cette conversion était essentiellement opérée par le produit du gène ATF2 (Cauet et al., 1999). Les levures représentent donc un organisme particulièrement utile sur le plan industriel pour la production de dérivés stéroïdiens.

Cependant, il est également connu que la 17α-hydroxyprogestérone peut, dans certaines conditions, être réduite par les levures en 4-prégnene-17α,20α-diol-3-one (Dumas et al., 1994) et que la production de ce sous-produit affecte les rendements de la synthèse ainsi que la qualité du produit final. Toutefois, à ce jour, la ou les activités enzymatiques responsables de cette réaction, de type 20α hydroxystéroïde déhydrogénase (20αHSD), n'ont pas été identifiées.

La présente invention résulte précisément de l'étude des activités endogènes de levure agissant sur l'hydroxyprogestérone et décrit l'identification de deux gènes codant des enzymes douées d'activité de type 20αHSD. Plus particulièrement, la présente demande montre que les gènes GCY1 et YPR1 sont porteurs de l'activité de type 20αHSD chez la levure, et que le produit de ces gènes permet par exemple de convertir l'hydroxyprogestérone en sous-produits in vitro. En effet, même si une inactivation du gène GCY chez la lavure a été décrite par Oechsner et al. (1988), aucun phénotype associé à cette inactivation n'a été identifié. De même, il a été proposé d'inactiver le gène YPR1 pour limiter la virulance des souches fongiques (WO99/25865, Microbia Inc.). La présente demande montre par ailleurs que la suppression de l'activité de ces gènes dans la levure réduit considérablement ou supprime la formation de sous-produits de type 4-prégnene-17α,20a-diol-3-one, et permet d'améliorer de manière significative les rendements de la synthèse de dérivés stéroidiens et/ou de transformer l'hydroxyprogestérone (ou ses précurseurs) en dérivés stéroidiens de manière plus sélective. La présente demande décrit donc de nouvelles compositions et méthodes utilisables pour la synthèse de dérivés stéroïdiens avec une meilleure sélectivité. L'invention décrit en particulier de nouvelles souches de levure ayant une activité de type 20αHSD réduite, et essentiellement incapables de transformer l'hydroxyprogestérone en sous-produits de type 4-prégnene-17α,20a-diol-3-one. L'invention peut également être utilisée pour augmenter la production de tels produits, en vue de leur utilisation ou transformation en composés actifs.

Un premier objet de l'invention réside plus particulièrement dans un procédé de modification d'un composé stéroïde, comprenant la mise en contact de ce composé (ou un précurseur de celui-ci) avec une levure présentant une activité 20αHSD réduite, en particulier une levure présentant un gène GCY1 et/ou YPR1 non fonctionnel, notamment disrupté, plus préférentiellement une levure du genre Saccharomyces, ou une préparation dérivée d'une telle levure.

L'invention concerne également l'utilisation d'une levure présentant une activité 20αHSD réduite, en particulier une levure présentant un gène GCY1 et/ou YPR1 non fonctionnel, notamment disrupté, plus préférentiellement une levure du genre Saccharomyces, ou une préparation dérivée d'une telle levure, pour la préparation, la production, la synthèse, la modification et/ou l'amélioration de composés stéroïdiens in vitro ou ex vivo.

L'invention concerne également tout procédé de production de dérivés stéroïdiens à partir de composés hydroxy-stéroide, notamment d'hydroxyprogestérone ou de ses précurseurs, utilisant une levure présentant une activité de type 20αHSD réduite, notamment une levure présentant un gène GCY1 et/ou YPR1 non fonctionnel, notamment disrupté, plus préférentiellement une levure du genre Saccharomyces, ou une préparation dérivée d'une telle levure.

L'invention concerne également un procédé de conversion de 17α-hydroxyprogestérone, notamment en 11-déoxycortisol, utilisant une levure présentant une activité de type 20αHSD réduite, notamment une levure présentant un gène GCY₁ et/ou YPR1 non fonctionnel, notamment disrupté, plus préférentiellement une levure du genre Saccharomyces, ou une préparation dérivée d'une telle levure.

L'invention a également pour objet l'utilisation d'une levure présentant une activité 20αHSD réduite, en particulier une levure présentant un gène GCY1 et/ou YPR1 non fonctionnel, notamment disrupté, plus préférentiellement une levure du genre Saccharomyces, ou une préparation dérivée d'une telle levure, pour la conversion de 17α-hydroxyprogestérone en 11-déoxycortisol.

Un autre objet de l'invention réside encore dans un procédé de modification de l'activité de type 20αHSD d'une levure, comprenant la modification de l'activité du gène GCY1 et/ou YPR1 de ladite levure. Il s'agit plus particulièrement d'un procédé pour réduire ou inhiber l'activité 20αHSD d'une levure, comprenant l'inactivation du gène GCY1 et/ou YPR1 de ladite levure, de préférence par disruption génique, encore plus préférentiellement sur des levures du genre Saccharomyces.

La présente invention a aussi pour objet des souches particulières de levure présentant une activité de type 20α-HSD réduite. Plus préférentiellement, il s'agit de levures possédant un gène YPR1 non fonctionnel, de levures possédant un gène GCY1 et un gène YPR1 non fonctionnels, ou encore de certaines levures possédant un gène GCY1 non-fonctionnel.

L'invention concerne aussi toute préparation acellulaire dérivée d'une levure telle que décrite ci-avant, notamment un lysat cellulaire, homogénat cellulaire, surnageant de culture, une solution (pré-)purifiée ou enrichie dérivée, etc.

Comme indiqué ci-avant, la présente invention décrit pour la première fois des souches (ou cellules ou cultures) de levure, ainsi que des préparations dérivées, présentant une activité de type 20αHSD réduite, voire indétectable. L'invention décrit en effet l'identification de gènes de levure porteurs de cette activité, les gènes GCY1 et YPR1, et montre que ces gènes peuvent être modifiés de manière spécifique, notamment au moyen des techniques de recombinaison génétique, sans nuire à la capacité de croissance ou de survie des cellules, ni à leur faculté de transformer ou convertir des composés stéroïdiens. L'invention fournit ainsi pour la première fois des procédés de synthèse, production, modification et/ou conversion de composés stéroïdes utilisant des levures avantageuses.

Un objet de l'invention réside donc plus particulièrement dans l'utilisation d'une souche (ou une cellule ou une culture) de levure caractérisée en ce qu'elle possède une modification génétique et en ce qu'elle présente une activité 20αHSD réduite, pour la production de composés stéroïdiens. La présente invention utilise plus particulièrement une souche de levure caractérisée en ce qu'elle possède :
- une modification génétique du gène GCY1, ou
- une modification génétique du gène YPR1, ou
- une modification génétique des gènes GCY1 et YPR1.

Plus préférentiellement, la ou les modifications génétiques présentes dans les levures de l'invention sont des modifications inactivatrices, c'est-à-dire conduisant à la perte d'activité du gène et/ou de la protéine correspondante. Un type tout particulièrement préféré de modification génétique inactivatrice selon l'invention est une disruption génique, comme il sera décrit en détails dans la suite du texte.

De manière plus spécifique, l'invention réside donc dans l'utilisation de levures dans lesquelles :
- le gène GCY1 est non-fonctionnel, ou
- le gène YPR1 est non-fonctionnel, ou
- les gènes GCY1 et YPR1 sont non fonctionnels, pour la préparation de composés stéroïdiens.

De telles levures présentent une activité 20αHSD réduite, voire indétectable, et sont donc particulièrement avantageuses pour la production ou la modification ou la conversion de composés stéroïdiens.

Selon un mode de réalisation préféré de l'invention, les levures appartiennent plus préférentiellement au genre Saccharomyces, notamment S. cerevisiae. Ainsi, dans un mode de mise en oeuvre plus spécifique, la présente invention réside dans des procédés ou utilisations de cellules (ou souches ou cultures) de levure du genre S.cerevisiae comprenant un gène GCY1 et/ou un gène YPR1 non-fonctionnel, de préférence disrupté.

Toutefois, bien que les exemples portent plus spécifiquement sur la levure Saccharomyces cerevisiae, il est entendu que l'enseignement de l'invention n'est pas limité à ce type particulier de levures et peut être étendu essentiellement à toute levure présentant une activité naturelle de type 20αHSD ou contenant un gène GCY1 ou YPR1. Dans ce contexte, on peut citer notamment les levures Saccharomyces, Kluyveromyces (notamment K. lactis), Schizosaccharomyces, Hansenula, Pichia (notamment P. pastoris), Candida (notamment C. maltosa), etc., dont la culture en fermenteurs et la modification génétique ont été décrites dans l'art antérieur.

D'autre part, au sens de l'invention, on entend par gène GCY1 le gène GCY1 de S.cerevisiae tel que décrit dans GenBank sous la référence X96740 (Bandlow et al., Gene 90(1), 1990, 105-114), ainsi que tout variant ou homologue fonctionnel de celui-ci présent dans les cellules de levure. De manière similaire, le gène YPR1 désigne le gène YPR1 (ou YDR368w) de S.cerevisiae tel que décrit dans GenBank sous la référence X80642, ainsi que tout variant ou homologue fonctionnel de celui-ci présent dans les cellules de levure. La séquence de ces gènes peut également être obtenue à partir d'autres banques dans lesquelles la séquence complète du génome de la levure S. cerevisiae est décrite (Université Stanford, MIPS, etc.). Les homologues fonctionnels peuvent être identifiés par recherche d'homologies de séquences, ou par clonage par hybridation, en utilisant des sondes dérivées des gènes GCY1 et YPR1 de S.cerevisiae, selon les techniques classiques de biologie moléculaire.

Comme indiqué, la présente invention réside dans des procédés ou utilisations de levures présentant une modification génétique d'un ou plusieurs gènes impliqués dans l'activité 20αHSD, notamment les gènes GCY1 et/ou YPR1, et ayant préférentiellement une activité 20αHSD réduite, voire supprimée.

Au sens de l'invention le terme « modification génétique » désigne toute altération du génome d'une cellule, obtenue par toute méthode possible, telle que l'emploi d'agents mutagènes et/ou la réalisation de modification(s) par voie génétique ou recombinante. Préférentiellement, une modification génétique est une modification de la séquence d'un gène au moins, résultant dans la modification de l'activité du gène, et notamment dans la stimulation ou, préférentiellement, l'inactivation dudit gène. L'inactivation d'un gène, ou le caractère non-fonctionnel d'un gène, peut se manifester par l'absence d'expression d'une protéine, par l'expression d'une forme non fonctionnelle de la protéine, en raison de mutation(s), délétion(s), substitution(s), insertion(s), etc., ou encore par l'expression de la protéine à des niveaux faibles, ne permettant pas une activité suffisante. De ce fait, la modification génétique d'un gène peut porter notamment sur tout ou partie de la région codante dudit gène ou d'une région régulatrice dudit gène (promoteur, etc.).

De manière préférée, la modification génétique selon l'invention comprend au moins une mutation, substitution, délétion et/ou insertion d'une ou plusieurs paires de bases dans la région régulatrice ou codante du gène considéré. Encore plus préférentiellement, il s'agit d'une modification par délétion de tout ou partie du gène considéré, qui peut être remplacé(e) par des séquences étrangères, selon la technique de disruption génique (ou « gêne replacement »). Les modifications génétiques par délétion et/ou insertion sont préférées pour la réalisation de la présente invention, dans la mesure où elles sont sélectives du gène considéré et stables dans le temps. Plus préférentiellement, la modification génétique réside donc dans le remplacement d'une partie au moins du gène considéré par des séquences étrangères. Cette modification peut être réalisée par des techniques connues consistant à préparer un gène modifié in vitro, qui peut être introduit dans le génome des levures par double recombinaison homologue, comme décrit dans les exemples (voir également Baudin et al., Nucleic Acids Res. 21 (14) (1993) 3329).

Ainsi, un objet préféré de l'invention réside dans des procédés ou utilisations de levures dans lesquelles tout ou partie du gène GCY1 et/ou YPR1 a été remplacé par des séquences étrangères (ou hétérologues), par exemple par un gène marqueur (codant une résistance à un antibiotique). Plus particulièrement, pour la disruption génique, un acide nucléique recombinant est préparé in vitro, comprenant une séquence étrangère choisie bordée de séquences homologues à des régions contigües ou non-contigües du gène considéré. La séquence étrangère peut être par exemple un gène marqueur, un gène complémentant une auxotrophié, un bloc d'expression, etc. Plus particulièrement, la séquence étrangère peut être un gène de sélection auxotrophe complémentant une exigence nutritionnelle de la souche de levure hôte, tel que le gène URA3, le gène LEU2, le gène TRP1, le gène HIS3 ou le gène ADE2 par exemple ; un gène de sélection dominant, tel qu'un gène de résistance à un antibiotique (G418, phléomycine, hygromycine B, etc.) ; ou encore un gène rapporteur (β-galactosidase, etc.). Il peut également s'agir d'un bloc interrupteur d'expression, comprenant par exemple un terminateur transcriptionnel tel que notamment un terminateur de levure choisi parmi CYC1, TDH3, TEF1 ou PGK. Il est entendu que toute autre séquence étrangère (i.e., non naturellement présente dans cette forme dans le gène considéré) permettant d'altérer les conditions d'expression du gène et/ou la structure même de la protéine codée peut être utilisée dans le cadre de la présente invention. L'acide nucléique ainsi préparé est ensuite introduit dans les levures, par les techniques conventionnelles (lithium, protoplastes, etc.), conduisant à l'insertion de la séquence étrangère dans le génome de la levure, au sein de la séquence du gène considéré, éventuellement en remplacement d'une région de celui-ci, par double recombinaison homologue.

Il est entendu que toute autre technique de modification génétique peut être utilisée dans le cadre de la présente invention, comme par exemple la mutagénèse dirigée, l'utilisation de transposons, etc.

Des exemples spécifiques de levures présentant un gène GCY1 et/ou YPR1 inactivé par disruption génique sont notamment :
- les cellules TGY170 (gcy1 ::LEU2) : dans les cellules TGY170, une partie du gène GCY1 a été remplacée par un acide nucléique codant pour la protéine LEU2 permettant la sélection des recombinants.
- les cellules TGY197 (gcy1 :LEU2, ydr368w ::URA3) : les cellules TGY197 comportent, par rapport aux cellules TGY170, une modification génétique supplémentaire affectant le gène YPR1 (également désigné YDR368w), dont une partie a été remplacée par le gène de sélection URA3.
- Les cellules TGY195 (ydr368w ::URA3) : les cellules TGY195 comportent une modification génétique affectant le gène YPR1 (également désigné YDR368W), dont une partie a été remplacée par le gène de sélection URA3.
- les cellules TGY194 (gcy1 ::URA3) : dans les cellules TGY194, une partie du gène GCY1 a été remplacée par un acide nucléique codant pour la protéine URA3 permettant la sélection des recombinants.

De telles cellules constituent également un objet particulier de l'invention. Notamment, l'invention concerne toute cellule (ou souche ou culture) de levure comprenant une modification génétique du (ou dans le) gène YPR1, notamment une délétion et/ou une insertion du ou dans le gène YPR1. L'invention concerne également toute cellule (ou souche ou culture) de levure comprenant une modification génétique des (ou dans les) gènes GCY1 et YPR1, notamment une délétion et/ou une insertion des ou dans les gènes GCY1 et YPR1. L'invention concerne aussi des préparations acellulaires dérivées de telles levures.

Les cellules de l'invention ou utilisées dans les procédés de l'invention présentent avantageusement une activité de type 20αHSD réduite, c'est-à-dire diminuée de 20% au moins, préférentiellement de 40% au moins, plus préférentiellement de 60% au moins, par rapport à la souche non modifiée génétiquement. Comme il est montré dans les exemples, l'invention démontre que l'inactivation du gène GCY1 dans la levure conduit à une réduction de 95% de l'activité de type 20αHSD dans le surnageant d'un homogénat cellulaire. Les résultats obtenus montrent également que la double modification génétique des gènes GCY1 et YPR1 conduit à la suppression de l'activité de type 20αHSD, qui est alors indétectable dans le surnageant d'un homogénat cellulaire. Ces résultats apportent la démonstration du rôle de ces gènes, et illustrent la possibilité de les modifier pour améliorer les propriétés des levures, pour les applications de production de dérivés stéroïdiens.

La présente invention est utilisable pour la production de composés stéroïdiens, en vue d'applications pharmaceutiques variées. A cet égard, l'invention décrit des méthodes de production de composés stéroïdiens utilisant les levures de l'invention. La demande réside également dans des méthodes améliorées de production de composés stéroïdiens utilisant des levures ayant une activité 20α-HSD réduite. Les méthodes de l'invention sont avantageusement réalisées par mise en contact, in vitro, d'une population de levures telles que décrites ci-avant avec un composé stéroïdien, suivie de l'extraction de composés synthétisés. Le composé stéroidien de départ peut être tout stéroïde naturel ou modifié ou de synthèse, notamment tout composé hydroxy-stéroïde ou précurseur, en particulier le cholestérol, la progestérone, la pregnénolone ou la 17OH-progestérone. Les procédés de l'invention sont utilisables pour la production de dérivés stéroidiens tels que le 11déoxycortisol, le cortisol, l'hydrocortisone, etc. ou des dérivés de ceux ci.

D'autres aspects et avantages de la présente invention apparaîtront à la lecture des exemples qui suivent, qui doivent être considérés comme illustratifs et non limitatifs.

### LEGENDE DES FIGURES

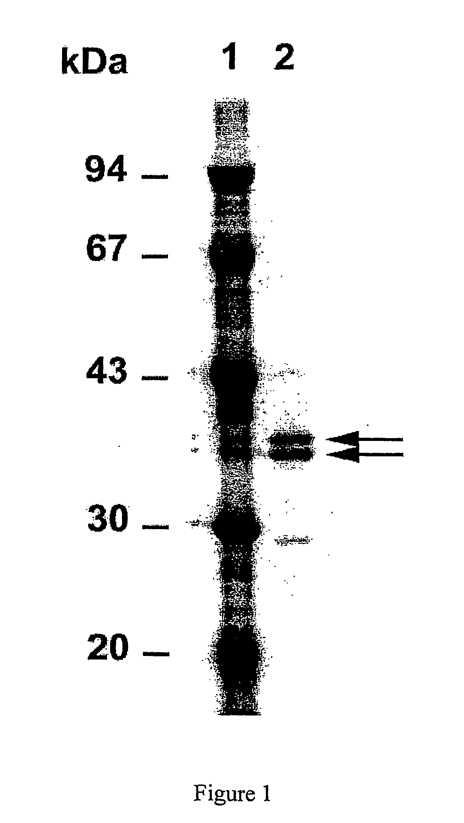

Figure 1 : Analyse SDS-PAGE d'une fraction purifiée de l'activité 20α-HSD par chromatographie sur Red120-Agarose à partir d'un homogénat de levure. La flèche supérieure indique la bande dont la séquence N-terminale correspond à GCY1, la flèche inférieure, celle correspondant à la séquence N-terminale de YPR1. La ligne 1 correspond au marqueur de poids moléculaire tandis que la ligne 2 correspond à la fraction purifiée de l'activité 20αHSD.

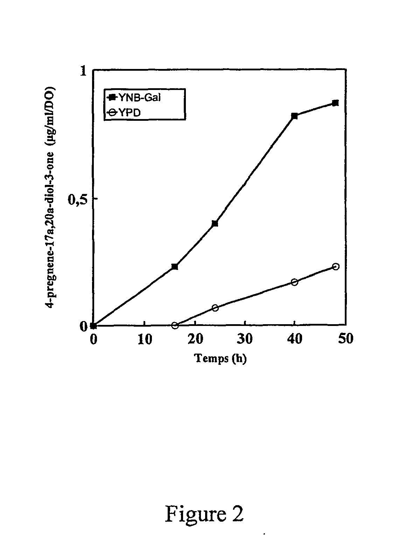

Figure 2 : Production de 4-pregnene-17α,20α-diol-3-one par des levures *S. cerevisiae* cultivées en milieu galactose (YNB-gal) ou glucose (YPD) en présence de 0,1mg/ml de 17αhydroxyprogesterone.

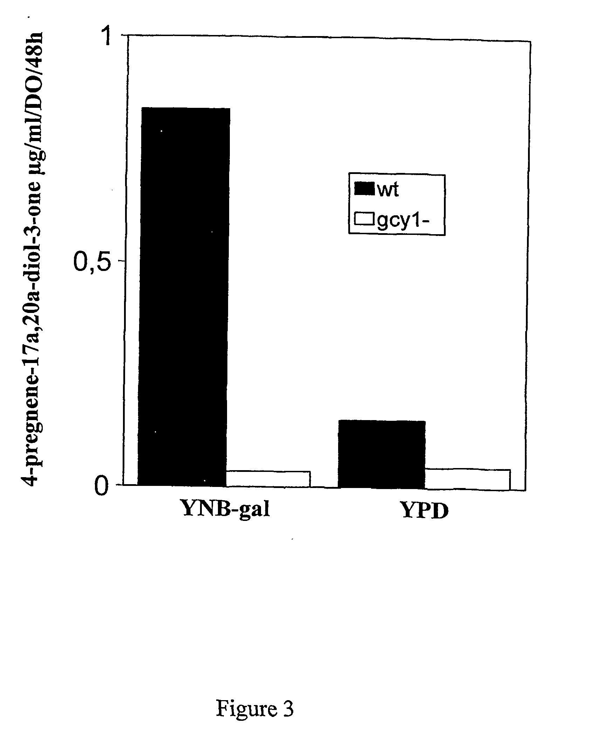

Figure 3 : Production de 4-pregnene-17α,20α-diol-3-one par des levures *S. cerevisiae* de type sauvage (wt) ou muté dans la séquence du gène *GCY1 (gcy-)* cultivées en milieu galactose (YNB-gal) ou glucose (YPD) en présence de 0,1mg/ml de 17αhydroxyprogesterone.

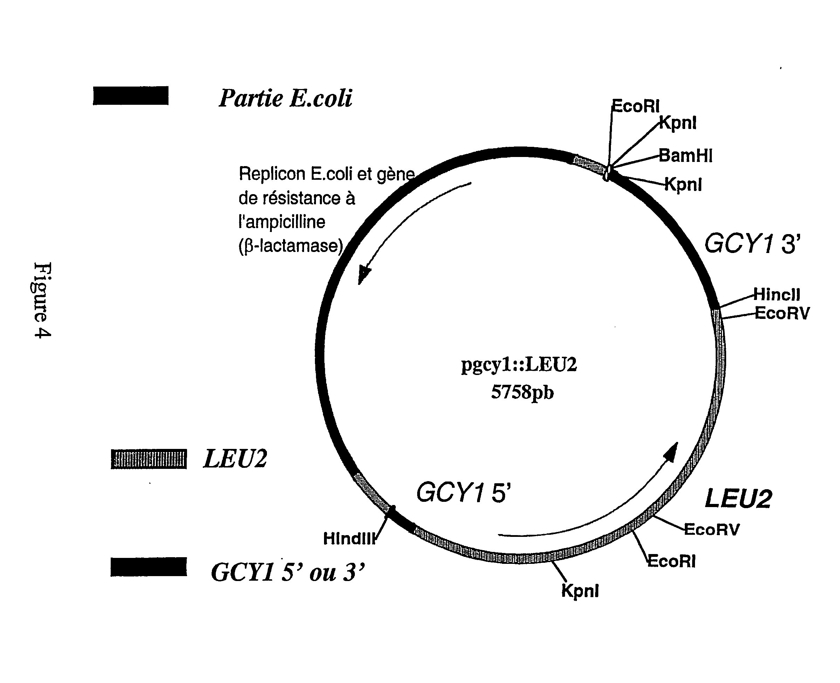

Figure 4 : Structure du plasmide de disruption du gène GCY1. Le plasmide est linéarisé par les enzymes BamHI et HindIII puis transformé dans S. cerevisiae selon la méthode décrite dans le paragraphe de Matériels et Méthodes. La délétion de la séquence du gène GCY1 comprend le promoteur et 306pb de séquence codante, y compris le codon de démarrage de la traduction.

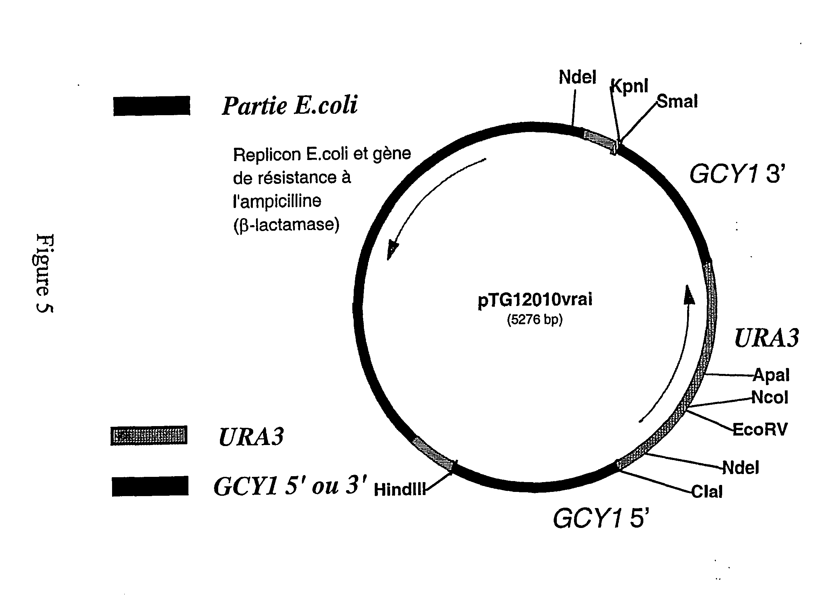

Figure 5 : Structure du plasmide de disruption du gène GCY1 (plasmide pTG12010 clone 40). Le plasmide est linéarisé par les enzymes EcoRI et SphI puis transformé dans S. cerevisiae selon la méthode décrite dans le paragraphe de Matériels et Méthodes. La délétion de la séquence protéique de GCY1 comprend les acides aminés 47 à 268 inclus. pTG12010 clone 36 possède la même structure, sans le site ClaI en 5' du gène URA3 mais avec un site HindIII en 3' du gène URA3.

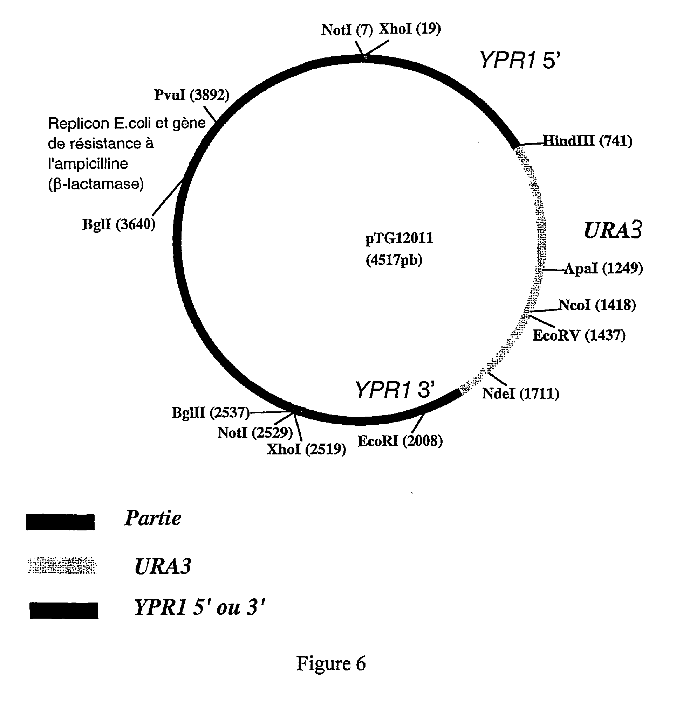

Figure 6 : Structure du plasmide de disruption du gène YPR1 (YDR368w) (plasmide pTG12011). Le plasmide est linéarisé par l'enzyme XhoI puis transformé dans S. cerevisiae selon la méthode décrite dans le paragraphe de Matériels et Méthodes. La délétion de la séquence protéique de YPR1 comprend les acides aminés 5 à 198 inclus.

### MATERIELS ET METHODES

Produits chimiques : La 17α-hydroxyprogestérone a été obtenue de Hoechst Marion Roussel (Romainville, France). Le Tergitol Nonidet P40 et le Tyloxapol proviennent de Sigma.

### Test enzymatique :

Conversion in vivo de 17α-hydroxyprogestérone : les cellules de levure ont été cultivées à 28°C en milieu YPD (10 ml) inoculé à A₆₀₀ = 0,1 à partir d'une préculture à 24 h. Ensuite, 100 µl d'une solution de 17α-hydroxyprogestérone (10 mg/ml) dans un mélange de Tergitol et d'éthanol (1:1; v:v) ont été ajoutés à la culture. Des aliquots du moût de culture (250 µl) ont été prélevés à différents intervalles et les stéroïdes ont été extraits avec du dichlorométhane. Les stéroïdes ont ensuite été séparés sur Ultrasphère ODS en présence de 45 % d'acétonitrile aqueux à un débit de 1 ml/min, à 45°C. ces stéroïdes ont été détectés à 240 nm.

### Cellules :

La souche E. coli BJ5183 (Hanahan, 1983) a été utilisée pour les recombinaisons in vivo et la souche C600, hsdR (Hubacek et Glover, 1970) pour les réactions classiques de ligation.

La souche parentale de levure FY1679-28c (MATa ura3-52 trpl-63 leu2- 1fen1 his3- 200 GAL) (Thierry et al., 1995) a été utilisée. Les souches TGY170, TGY197, TGY195, TGY194 TGY212, TGY245 et FY1679-28c/pTG10497 ont été construites comme décrit dans les exemples.

Les méthodes classiques de biologie moléculaire et de recombinaison in vivo chez E. coli ou dans la levure ont été utilisées, comme décrit dans Sambrook et al. (1989) ou dans Degryse et al (1995, 1996).

### Culture des levures :

Les levures ont été cultivées généralement sur milieu minimum synthétique (Sherman, 1991) supplémenté d'apports nutritionnels à 100 µg/ml. Pour les transformations de S. cerevisiae, les cellules ont été rendues compétentes selon la technique à l'acétate de Lithium (Ito et al., 1983), après croissance en milieu YPD (Sherman, 1991).

### RESULTATS

### Identification de l'activité 20α-HSD de levure, responsable de réactions parasites sur la 17α-hydroxyprogestérone

La réduction NADPH dépendante de la 17α-hydroxyprogestérone sur le C20 en 4-prégnene-17α,20α-diol-3-one par la levure S. cerevisiae a été décrite précédemment (Dumas et al., 1994). Cette activité est similaire à l'activité 20α-HSD rapportée dans différents tissus. Les enzymes caractérisées à partir de ces tissus sont monomériques, avec un poids moléculaire de 35 kDa environ. Dans le but d'identifier la ou les enzymes responsables de l'activité 20α-HSD chez la levure, des recherches d'homologies avec l'enzyme 20α-HSD de testicule bovin dans les banques de S. cerevisiae ont été réalisées. Ces recherches ont permis d'identifier 6 produits de gènes de levure présentant 44 à 32 % d'identité de séquences d'acides aminés avec l'enzyme de mammifère. Ces gènes sont rassemblés dans le Tableau I.

Dans le but de mieux caractériser les enzymes impliquées, l'activité de type 20α-HSD de levure a été reconstituée in vitro en utilisant la 17α-hydroxyprogestérone et le NADPH comme substrats. Différentes préparations dérivées de culture de levure S. cerevisiae ont été testées dans ce système, ce qui a permis la localisation de l'activité dans le surnageant après centrifugation à 100 000 xg d'un homogénat cellulaire. Ce résultat indique que l'activité enzymatique est soluble. Une purification partielle de l'activité de type 20α-HSD par chromatographie Red 120 a ensuite été réalisée, ce qui a permis l'obtention d'un doublet dans la région 35 kDa, après SDS-PAGE (Fig. 1). Le séquençage de ces bandes a montré qu'elles étaient composées principalement du produit des gènes GCY1 et YPR1. Ces deux enzymes font partie des homologues bovines de 20α-HSD listées dans le Tableau I. La séquence complète des gènes GCY1 et YPR1 est accessible par exemple dans GenBank sous les références X96740 et X80642 respectivement.

Il est intéressant de noter que GCY1 a été décrit comme codant une aldo-kéto-réductase (AKR) dont l'expression est significativement accrue en présence de galactose (Magdolen et al., Gene 90(1), 1990, 105). Les enzymes AKR ont une large spécificité de substrat. Elles métabolisent différents substrats, incluant les aldéhydes aliphatiques, monosaccharides, prostaglandines, et stéroïdes. Ainsi, GCY1 constitue un bon candidat potentiel, et nous avons décidé de vérifier si cette enzyme pouvait être impliquée dans la génération de 4-prégnène-17α-20α-diol-3-one à partir de 17α-hydroxyprogestérone.

### Caractère inductible de l'activité 20α-HSD et expression en système acellulaire

Les expériences réalisées ont permis de montrer que l'activité 20α-HSD est inductible par le galactose dans la levure. Ainsi, la conversion in vivo de 17α-hydroxyprogestérone en 4-prégnène-17α-20a-diol-3-one a été déterminée dans des cultures de levures cultivées sur différentes sources de carbone. Le délai observé quand les levures sont cultivées sur du glucose n'est pas observé en présence de galactose (Fig. 2). Cette observation est en accord avec une répression par le glucose du gène codant 20α-HSD. La conversion débute après 16 h lorsque le glucose est épuisé. La conversion de 17α-hydroxyprogestérone en 4-prégnène-17α-20α-diol-3-one est environ 4 fois plus importante après 48 h lorsque les levures sont cultivées en présence de galactose. Ces résultats ont par ailleurs été confirmés in vitro en mesurant l'activité 20α-HSD sur un extrait acellulaire obtenu à partir de levures cultivées en milieu galactose ou glucose. Les activités spécifiques 20α-HSD étaient respectivement de 0,05 et de 0,75 µM/min/mg dans les homogénats de cellules cultivées en milieu glucose ou galactose.

Ces résultats montrent donc (i) que l'activité 20α-HSD est portée par le produit des gènes GCY1 et YPR1 de levure, (ii) que ces enzymes sont solubles, et (iii) que leur activité peut être augmentée en présence de galactose et réprimée en présence de glucose.

### Construction et propriétés de levures contenant un gène GCY1 et/ou YPR1 non fonctionnel

Dans le but de confirmer que Gcylp était responsable de l'activité 20α-HSD, le gène correspondant de ORF YOR120w a été délété ("Knock Out") du génome de la levure. Les résultats obtenus montrent que la souche obtenue présente une activité 20α-HSD fortement réduite par rapport à la souche sauvage. Par ailleurs, des souches dans lesquelles le gène YPR1 seul ou en combinaison avec GCY1 est délété ont également été construites et testées pour leur activité, comme décrit ci-après.

### . Construction des levures GCY1 et/ou YPR1 déficientes :

Les levures déficientes en activité GCY1 et/ou YPR1 ont été préparées par disruption génique. Plus particulièrement :
La souche TGY170 (FY1679-28c, gcy1::LEU2) a été construite par disruption du gène GCY1 au moyen du plasmide Pgcy1::LEU2.
La souche TGY197 (FY1679-28c, gcy1::LEU2 ydr368w::URA3) a été générée par disruption supplémentaire du gène YDR368w (YPR1), selon la méthode décrite pour ATF2 (Cauet et al., 1999) au moyen du plasmide pTG12011.
Les souches TGY195 ont été générées par disruption du gène YDR368w (YPR1), selon la méthode décrite pour ATF2 (Cauet et al., 1999) au moyen du plasmide pTG12011.
Les souches TGY194 (FY1679-28c, gcyl::URA3) ont été construites par disruption du gène GCY1 au moyen du plasmide pTG12010.
Les souches FY1679-28c/pTG10497 et TGY245 ont été construites au moyen des plasmides pTG10497 et pTG12045.

Les plasmides suivants ont été utilisés pour disrupter les gènes GCY1 et YPR1 : pgcy1::LEU2, pTG12010, pTG12011 (Figures 4-6), pTG12086 et pTG12045. Le plasmide monocopie pTG10497 est utilisé pour l'expression de la P450c21.

Le plasmide pgcy1 : :LEU2, décrit par Magdolen et al Gene, 90 (1990) 105-114, contient le gène GCY1 dont la séquence codante et le promoteur ont été interrompus par la séquence codant pour le gène LEU2. Plus précisement, le promoteur et la partie codante correspondant au fragment de restriction EcoRV, HincII ont été remplacés par le fragment HpaI de 2,17 Kbases du gène LEU2. Ainsi le gène GCY1 a été délété de son promoteur et de 306 paires de bases de sa séquence codante.

Le plasmide pgcyl : : LEU2 a été linéarisé par les enzymes de restriction HindIII et BamHI, le fragment HindIII BamHI de 3.1kb contenant le gène disrupté a été préparé pour transformer la souche Fy 1679-28c en utilisant le protocole décrit par Gietz RD et al (Yeast 1995 Apr 15;11(4):355-60 Studies on the transformation of intact yeast cells by the LiAc/SS-DNA/PEG procédure). Les colonies ont été sélectionnées sur un milieu dépourvu en leucine. Les colonies positives dans ce crible sont ensuite cultivées en milieu riche pour faire une bioconversion de 17OH progestérone comme décrit dans Dumas et al (Eur J Biochem 1996 Jun 1;238(2):495-504 11 beta-hydroxylase activity in recombinant yeast mitochondria. In vivo conversion of 11-deoxycortisol to hydrocortisone). La concentration du substrat 17OH progestérone est de 100mg/l, la source de carbone est le galactose et la densité optique de départ est de 0,1. Le volume de la culture est 10ml, l'incubation est de 48 heures à 30°C. Après 48 heures d'incubation, les clones positifs sont évalués par extraction d'un ml de milieu (avec les cellules) avec 2ml de dichlorométhane puis analyse de la phase organique par chromatographie haute pression en phase reverse comme décrit ci-dessus (Dumas et al 1996). Les chromatogrammes sont analysés pour la présence del7 20 dihydro progestérone en comparaison avec du produit purifié. Lors de cette incubation, il apparaît dans le milieu de culture de la souche sauvage (non transformée par le fragment de disruption) une quantité de l'ordre de 4mg/l de 17, 20 dihydro progestérone soit 4% du substrat, tandis que dans certains transformants la présence de 17, 20 dihydroprogestérone n'est plus que 1mg/l. Une souche TGY170 convertissant à un faible niveau la 17OH progestérone en 17,20 OH progestérone et présentant une croissance identique à la souche sauvage est sélectionnée.

Deux nouveaux plasmides pTG12010 et pTG12011 ont été construits pour permettre la disruption des gènes GCY1 et YPR1 associés au marqueur de sélection URA3.

Le plasmide pTG 12010 a été construit sur une base du plasmide pUC19 (Gene 1985;33(1):103-19 Improved M13 phage cloning vectors and host strains: nucleotide séquences of the M13mp18 and pUC19 vectors. Yanisch-Perron C, Vieira J, Messing J) tandis que le plasmide pTG12011 a été construit sur une base du plasmide pPOLYIII (Lathe,R., Vilotte,J.-L. and Clark,J.A. Plasmid and bacteriophage vectors for excision of intact inserts JOURNAL Gene 57, 193-201 (1987)).

### Construction des plasmides pTG12010 et pTG12011

La construction de la disruption du gène GCY1 par le gène URA3 dans le plasmide pUC19 pour aboutir à pTG12010 a été obtenue par quatre amplifications par PCR successives. D'une part trois PCR indépendantes ont été réalisées pour obtenir la partie 5' du gène GCY1 (PCR1), le gène URA3 fonctionnel bordé par des séquences GCY1 (PCR2), la partie 3' du gène GCY1(PCR3). Les parties 5' et 3' du gène GCY1 ont été obtenues à l'aide des couples OTG11285 , OTG11286 et OTG11287, OTG11289, sur une matrice d'ADN génomique de la souche Fy 1679-28c. La séquence des oligonucléotides est la suivante : OTG11285 : GATTCGGTAATCTCCGAACAggtaccAATTATATCAGTTATTACCCGGGA (SEQ ID NO 1); OTG11286 : AGCCATCTTTCAAAGCGGTT (SEQ ID NO : 2) ; OTG11287 : CCGATCGAATCAAAACGAACAG (SEQ ID NO : 3) ; OTG11289 : TCTAATCAGCTAGTAAGAAC (SEQ ID NO : 4).

Le gène URA3 flanqué de séquences GCY1 (de tel façon à obtenir une délétion d'une partie de la séquence codante du gène GCY1) est amplifié à l'aide des oligonucléotides OTG 11305 (aaccgctttgaaagatggctATCGATTTTCAATTCAATTCATCATTTTTTTTTTATTCTT TTTTTTG, (SEQ ID NO : 5) et OTG11306 (ctgttcgttttgattcgatcgggAAGCTTGGGTAATAACTGATATAATTAAATTGAACTC (SEQ ID NO : 6) à partir d'une matrice plasmide pTG10054 linéarisé (Degryse et al In vivo cloning by homologous recombination in yeast using a two-plasmid-based system. Yeast. 1995 Jun 15;11(7):629-40). Les conditions de tampon et de concentration en matrice et amorces pour l'amplification sont décrites par le producteur ou fabricant de l'enzyme TAQ DNA polymerase, et en particulier pour l'enzyme elongase développée par Life Technologies. Les cycles de températures sont les suivants : un premier cycle de 6'30 pour dénaturer amorce et matrice puis 30 cycles de 30s à 93°C, 2 mn à 54°C et 3mn à 68°C , le dernier cycle est de 5 mn à 72°C. Les produits PCR1, PCR2 et PCR3 sont mélangés de façon equimoléculaire et amplifiés de nouveau à l'aide des oligonucléotides OTG 11285 et OTG11289 (Voir ci dessus). Le produit final PCR4 d'une taille d'1,9 Kbases est ensuite sous cloné entre les sites de restriction KpnI et BamHI du plasmide pUC19 pour obtenir le plasmide pTG12010. La structure du plasmide a été vérifiée par profil de restriction et séquencage nucléotidique des extrémités. Le clonage du pTG12010 a permis en fait d'obtenir deux versions de ce plasmide, la version pTG12010#40 (pTG12010 clone 40) et pTG12010#36 (pTG12010 clone 36). La volonté initiale était d'obtenir le gène GCY1 interrompu par le gène URA3 bordé par les sites de ClaI et HindIII respectivement en 5' et en 3' du gène. En fait deux plasmides différents ont été obtenus le PTG12010#36 et pTG12010 #40. Ces deux plasmides différent uniquement par la présence ou l'absence de sites ClaI et HindIII aux extrémités du gène URA3. Le plasmide pTG12010#40 possède un site de restriction HindIII à l'extrémité 3' du gène URA3 mais pas de site ClaI en 5'. Le plasmide pTG12010 #36 ne possède pas de site HindIII à l'extrémité 3' mais un site ClaI à l'extrémité 5' du gène.

Cette propriété est utilisée pour obtenir le plasmide qui possède le gène URA3 bordé par les sites HindIII et ClaI interrompant la séquence codante de GCY1.

### Construction du plasmide pTG12036.

Le plasmide pTG12036 a été construit en 4 étapes à partir du pTG10802. Le plasmide pTG10801 (qui est à l'origine du plasmide pTG10802) est un plasmide de type pUC dans lequel une suite de sites de restriction a été insérée entre les sites XhoI et XhoI. Cette suite de sites comprend les sites HindIII, SnabI, ClaI et SpeI. Entre les sites HindIII et

ClaI, la cassette HindIII ClaI du pTG10470 (comme décrit ci-après) comprenant le promoteur TEF1, le cDNA P450c21 humain et le terminateur PGK a été insérée entre les sites HindIII et ClaI de pTG10801 pour donner pTG10802. Ce plasmide a été ensuite digéré par XhoI et donc la cassette introduite est délétée pour introduire un fragment de PCR bordé par des sites XhoI. Ce fragment de 2,5kb provient de l'amplification par la paire d'oligonucléotides OTG11844. (tttgctcgaggttacagaagggc, SEQ ID NO : 13) et OTG11845 (gattctcgagcaattggctgacta, SEQ ID NO : 14) sur le plasmide pTG12010 (#40) pour obtenir un fragment bordé par des XhoI contenant le gène GCY1 interrompu par le gène URA3 bordé en 5' par un site de restriction ClaI. Ce fragment a été cloné entre les sites XhoI du plasmide pTG10802 pour obtenir le plasmide pTG12035. Dans le but d'introduire le site HindIII manquant, le plasmide pTG12010 (# 36) a été utilisé. Ce plasmide est essentiellement identique au pTG12010 (# 40) mais possède un site HindIII en 3' du gène URA3 à la limite avec le gène GCY1 et ne possède pas de site ClaI en 5' du gène URA3 à la jonction avec le gène GCY1. On procède à une recombinaison in vivo dans E. coli, entre le fragment NcoI BamHI de 2,2 kb de pTG12010 (# 36) (qui porte de 5' vers 3' un fragment du gène URA3 et en 3' un fragment du gène GCY1) et une partie du plasmide pTG12035 c'est-à-dire le grand fragment StuI, AflII de 4,45kb. Le plasmide obtenu pTG12036 possède le gène GCY1 interrompu par le gène URA3 bordé par des sites ClaI et HindIII respectivement en 5' et 3'.

### Construction du plasmide pTG12086

Ce fragment est ensuite remplacé par la cassette d'expression de la P450c21 portée par le fragment 2,33Kb ClaI, HindIII du plasmide pTG10469 ( voir plus bas) pour obtenir le plasmide pTG12036.

### Construction des plasmides d'expression pour le cytochrome P450c21.

Pour la surexpression de cette protéine dans la levure deux types de promoteur ont été utilisés, TEF1 (« Transcription elongation factor1 ») et TDH3 (« Glyceraldehyde-3-phosphate dehydrogenase 3). Dans tous les cas, le terminateur de transcription est le terminateur PGK.

Dans ces plasmides, le fragment SalI, MluI porte le cDNA de la P450c21 humaine.

### Construction des plasmides pTG10470 et pTG10469..

Le plasmide pTG10289 a été obtenu par modification du pMAc21 (Expression and functional study of wild-type and mutant human cytochrome P450c21 in Saccharomyces cerevisiae.

Wu DA, Hu MC, Chung BC DNA Cell Biol 1991 Apr; 10(3) :201-9)) digestion KpnI, MluI et introduction de l'oligonucléotide OTG5868. Le cDNA de ce plasmide provient de l'American Type Culture Collection sous le nom de pc21/3c. C'est le fragment EcoRI-BamHI de 1,6Kb qui a servi de base pour la construction des différents plasmides. Les modifications apportées sont décrites dans l'article ci-dessus et dans l'article (Expression of human 21-hydroxylase (P450c21) in bacterial and mammalian cells : A system to characterize normal and mutant enzyme Meng-Chun Hu and Bon-chu Chung DNA and Cell Biology 1991 Apr; 10 (3) 201-209)

Dans cette manoeuvre, la partie non codante de la P450c21 du plasmide pMAc21 qui contient la cassette d'expression de la P450c21 a été éliminée ainsi que le site KpnI qui s'y trouvait. Le plasmide pTG10292 a été obtenu par transfert du cDNA c21 humain (fragment SalI, MluI)du plasmide pTG10289 dans le plasmide pTG10031 à l'aide des sites SalI et MluI. Le plasmide pTG10475 a été obtenu par PCR et recombinaison. En effet à partir du plasmide pTG10292, un fragment du cDNA P450c21 humain représentant approximativement 250 nucléotides a été amplifié à l'aide des oligonucléotides OTG7410 (GGAATTCCGTCGACAAAAATGCTGCTCCTGGGCCTGCTGC, SEQ ID NO :15) et OTG5927 (CCTCAATGGTCCTCTTGGAGTTCAGCACC, SEQ ID NO :16). Ce fragment représente la séquence codante de la P450c21 humaine bordé d'un site SalI et de la séquence AAAA, comme décrit dans l'oligonucléotide OTG7410. Ce fragment a été digéré par SalI puis ligué sur le fragment linéaire de pTG10292 digéré par SalI et ensuite une expérience de recombinaison a été réalisée dans la souche BJ5183. Le plasmide obtenu pTG10475 porte un cDNA de la P450c21 avec une séquence codante identique à celle du cDNA naturel contrairement au plasmide pMAc21 sur un fragment qui est compatible avec les vecteurs que nous utilisons au laboratoire, c'est à dire un fragment bordé par les sites de restriction SalI et MluI. Ce fragment possède l'environnement suivant autour du codon ATG d'initiation de la traduction GTCGACAAAAATGCTGCTCCTGGGCCTGCTGC (SEQ ID NO :17). A partir de ce plasmide le fragment SalI, MluI portant le cDNA de la P450c21 humaine a été transféré dans le plasmide pTG10158 (Degryse et al 1996) par clonage classique pour donner le plasmide pTG10472. Ce même fragment SalI MluI du plasmide pTG10472 a été ensuite transféré par recombinaison dans le plasmide pTG 10085 (Degryse et al 1996) pour donner le plasmide pTG 10469. Ce même fragment portant le cDNA P450c21 sur un fragment de restriction SalI et MluI a été transféré dans le plasmide pTG10092 pour donner le plasmide pTG10470 ( Degryse et al 1996). Ce plasmide porte donc le cDNA de la P450c21 humaine sous contrôle du promoteur TEF1 et d'un terminateur PGK avec un marqueur de sélection URA3-d avec un environnement du codon initiateur ATG, comme décrit précédemment.

### Construction du plasmide pTG12086.

Ce plasmide sert à l'intégration d'une cassette d'expression pour la P450c21 ainsi qu'à la disruption du gène GCY1 dans le même temps.

Ce plasmide a été construit à partir du plasmide pTG12036 et du plasmide pTG10614.

Ce dernier plasmide a été construit à partir du pTG10212 ( Degryse et al Yeast 11 : 629-640 (1995)) qui est un plasmide d'expression levure basé sur un promoteur TDH3, un terminateur PGK et un marqueur de sélection URA3-d.

Par recombinaison homologue dans *E.coli,* le marqueur de sélection est remplacé par le marqueur de sélection du plasmide pTG10054 (Degryse et al 1995), pour ce faire le fragment MluI, FspI du pTG10054 de 2,1 kb contenant le marqueur URA3 flanqué par des séquences de recombinaison est recombiné avec le grand fragment HindIII de pTG10212 pour donner le plasmide pTG10610 qui possède les mêmes caractéristiques que pTG10212 (Degryse et al 1995) avec un marqueur URA3 dans la même orientation que pTG10054. Le fragment SalI MluI portant le cDNA du cytochrome P450 c21 humain du plasmide pTG10472 (voir plus haut) est transféré dans le plasmide pTG10610 pour donner le plasmide pTG10614. Le fragment ClaI HindIII de ce plasmide contenant de 5' vers 3', le promoteur TDH3, le cDNA de la P450c21 humaine bordé par des sites SalI et MluI puis le terminateur PGK est transféré dans le plasmide pTG12036 pour donner le plasmide pTG12086 qui contient donc la séquence du gène GCY1 interrompue par la cassette d'expression TDH3 du cytochrome P450c21 humain.

### Construction du plasmide pTG12045.

Le site unique SphI du plasmide pPolyIII est détruit par insertion de la paire d'oligonucléotides complémentaires OTG11975 (AAATCGATAACATG, SEQ ID NO :18) et OTG 11976 (TTATCGATTTCATG, SEQ ID NO :19). Le site SphI du pPOLYIII est détruit et remplacé par un site ClaI pour donner le plasmide pTG12040. Dans le plasmide pTG12040 entre les sites uniques ClaI et EcoRI on introduit un fragment d'ADN génomique ClaI EcoRI correspondant à la partie 3' de 0.7kb du gène YPR1 obtenu par amplification avec les oligonucléotides OTG11981 (ATTGATATCGATAAAAAGCACGGCGTTGAG, SEQ ID NO :20) et OTG11982 (TCTCGGAATTCAGGTACTGCAGCCAG, SEQ ID NO :21) pour donner le plasmide pTG12041. Dans ce plasmide pTG12041 de 2,84kb, la partie 5' du gène YPR1 (0.66kb) amplifiée par les oligonucléotides OTG11314 (tacgctcgagACGTTGGTGTCATTGATATTCA, SEQ ID NO :22) et OTG11980 (CAACTAAGCTTCATTCAAATAGATAGCCGC, SEQ ID NO :23) à partir d'ADN génomique de levure sauvage est clonée sous forme d'un fragment XhoI HindIII entre les sites SalI et HindIII du plasmide pTG12041. On obtient le plasmide pTG12042 de 3,5kb. Ce plasmide porte le gène YPR1 interrompu par les sites ClaI et HindIII. Entre ces sites la cassette du cytochrome P450c21 est clonée sous forme d'un fragment ClaI HindIII de 2,33 kb provenant du plasmide pTG10469. On obtient ainsi le plasmide pTG12045.

### Construction du plasmide pTG10497.

Ce plasmide est un plasmide d'expression à faible nombre de copie (de type ARS CEN), qui contient une cassette d'expression pour le cytochrome P450c21 humain qui se retrouve sous contrôle du promoteur TDH3 et du terminateur PGK. Ce plasmide a été construit à partir du plasmide pTG10434 qui contient le marqueur de sélection URA3 ainsi que le promoteur TEF1, le terminateur PGK ainsi qu'une origine de réplication dans la levure du type ARSH4/CEN6 (Degryse et al 1995) .

Ce plasmide a été modifié de façon à contenir un marqueur LEU2 et promoteur TDH3 à la place des marqueurs URA3 et promoteur TEF1, respectivement. Pour ce faire le fragment SpeI, MluI qui contient le marqueur LEU2 bordé par les fragments de recombinaison qui sont le terminateur PGK et un fragment de l'origine de réplication est cloné par recombinaison à la place de la région URA3 du plasmide pTG10434 digéré par HindIII pour obtenir le plasmide pTG10466. Dans ce plasmide pTG10466, le promoteur TEF1 est remplacé par le promoteur TDH3 en recombinant dans E.coli le fragment HindIII-EcoRI de pTG10212 (Degryse et al 1995) (contenant l'origine de réplication de E.coli ainsi que le promoteur et le terminateur TDH3 et PGK respectivement) avec le fragment MluI FspI de pTG10466 qui contient le marqueur LEU2 et l'origine de réplication ARSCEN bordés par les séquences de recombinaison; on obtient ainsi le plasmide pTG10612. Entre les sites SalI et MluI de ce plasmide, la cassette d'expression TDH3 : :cytochrome P450c21 humain et terminateur est placée pour donner le plasmide pTG10497.

### Construction des souches déficientes

Pour obtenir la souche dépourvue d'activité GCY1, la souche FY 167928c est transformée par le plasmide pTG121010 linéarisé par les enzymes SphI et EcoRI selon la méthode à l'acétate de lithium décrite précédemment. Les clones transformés sont sélectionnés sur un milieu dépourvu en uracil puis criblés par amplification sur colonie à l'aide des paires d'oligonucléotides OTG11285 , OTG11289 et

OTG11285, OTG11306 en utilisant comme matrice un extrait ou une préparation d'ADN génomique de levure selon les conditions décrites ci-dessus. Les colonies qui montrent des produits ADN de PCRs de la taille attendue respectivement 1,9Kb et 1,4Kb sont ensuite cultivées en milieu riche pour faire une bioconversion de 17OH progestérone comme décrit dans Dumas et al (Eur J Biochem 1996 Jun 1;238(2):495-504 11 beta-hydroxylase activity in recombinant yeast mitochondria. In vivo conversion of 11-deoxycortisol to hydrocortisone). La concentration de substrat est de 100mg/l, la source de carbone est le galactose et la densité optique de départ est de 0,1. Le volume de la culture est 10ml, l'incubation est de 24 heures à 30°C. Après 24 heures d'incubation, les clones positifs sont évalués par extraction d'un ml de milieu ( avec les cellules) avec 2ml de dichlorométhane puis analyse de la phase organique par chromatographie haute pression en phase réverse comme décrit ci-dessus (Dumas et al 1996). Les souches TGY194 #10 et TGY194 #11 sont dépourvues d'activité 20 keto reductase sur la 17OH progestérone et donnent un signal positif en PCR.

La construction de l'interruption du gène YPR1 (YDR368w) par le gène URA3 dans le plasmide pPOLYIII pour aboutir à pTG12011 a été obtenue par 4 PCR successives. D'une part trois PCR indépendantes ont été réalisées pour obtenir la partie 5' du gène YPR1 (PCR 5), le gène URA3 fonctionnel bordé par des séquences YPR1 (PCR 6), la partie 3' du gène YPR1 (PCR 7). Le DNA de la PCR5 a été obtenu par amplification sur une matrice d'ADN génomique avec les oligonucléotides OTG11314 (tacgctcgagACGTTGGTGTCATTGATATTCA, (SEQ ID NO: 7) et OTG11315 (CTTCATTCAAATAGATAGCCG, (SEQ ID NO : 8), de même le DNA de la PCR7 est obtenu par amplification à l'aide des oligonucléotides OTG11316 (TATGGCTAAAAAGCACGGCGTT, (SEQ ID NO : 9) et OTG11317 (cgatctcgagTTTCTCGTTGTTCAGGTACTG, (SEQ ID NO: 10) sur la même matrice. Le gène URA3 flanqué de région YPR1 5' et 3'est amplifié à l'aide des oligonucléotides OTG11463 (CGGCTATCTATTTGAATGAAGatcgattttcaattcaattcatcattttttttttattct tttttttg, (SEQ ID NO: 11) et OTG11464 (AACGCCGTGCTTTTTAGCCATAAGCTTgggtaataactgatataattaaattgaactc, (SEQ ID NO :12) sur une matrice pTG10054 linéarisée comme décrit ci dessus. Les produits des PCRS, PCR6 et PCR7 ont été mélangés de façon équimoléculaire puis amplifiés par PCR à l'aide des oligonucléotides OTGI1314 et OTG11317 pour obtenir un produit de PCR8 de 1,X kb comme décrit précédemment. Ce produit PCR8 a été digéré par l'enzyme XhoI puis sous cloné dans le plasmide pPOLYIII digéré par XhoI. L'orientation de l'insertion dans le plasmide pPOLYIII a été déterminée par digestion par les enzymes NcoI et EcoRI.

Curieusement, on note l'absence d'un site ClaI et d'un site HindIII respectivement pour le plasmide pTG12010 et pTG12011. Les jonctions de clonage ont été vérifiées par séquencage nucléotidique.

### Construction de la souche TGY195.

Le plasmide pTG12011 est digéré par l'enzyme XhoI, le produit de la digestion puis utilisé pour transformer la souche Fy 1679-28c en utilisant la méthode au chlorure de lithium précédemment citée. Les transformants sont sélectionnés sur un milieu dépourvu en uracil. Les transformants sont analysés par amplification par PCR en utilisant les oligonuclétides qui ont servi à la construction du plasmide pTG12011. Les clones positifs dans ce test sont ensuites criblés par la méthode de bioconversion du 17OH progestérone décrite plus haut en présence de glucose comme source de carbone. Un clone TGY195#4 est sélectionné pour de nouvelles caractérisations.

### Construction de la souche TGY197

Un clone TGY195#4 présentant une activité 20 keto reductase réduite dans ces conditions est sélectionné pour une nouvelle transformation à l'aide du plasmide pgcy1 : :LEU2 comme décrit précédemment pour la souche Fy1679-28c. Les clones capables de croître en l'absence de Leucine sont ensuite sélectionnés pour une nouvelle bioconversion sur la 170H progestérone comme décrit précédemment en présence de glucose ou de galactose. Un clone TGY197#a présentant une activité réduite dans les deux conditions de bioconversion est selectionné. Ainsi l'activité 20 keto reductase qui à l'origine était de 12% (à 100mg/l de substrat) est réduite à 0.2% environ soit une réduction de plus de 60 fois.

### Construction de la souche TGY245.

La souche TGY245 est construite à partir de la souche TGY195#4. A partir de la souche TGY195#4, on obtient d'abord la souche TGY212#1 puis la souche TGY243#1 et enfin la souche TGY245.

La souche TGY195#4 est transformée à la fois par le plasmide YRP7 (1µg) et 5µg de plasmide pTG12045 digéré par NotI. Les souches transformées sont sélectionnées sur un milieu dépourvu en tryptophane. Des colonies (678 colonies) sont repiquées sur un milieu contenant du tryptophane (pour se débarrasser du plasmide YRP7) et sur un milieu contenant du tryptophane et du 5 fluoro orotate (5FO) pour sélectionner les colonies qui ont perdu le gène URA3 interrompant le gène GCY1. Une colonie est sélectionnée dans ce crible, TGY212#1. Cette colonie est soumise à une expérience de bioconversion comme décrit précédemment avec 100µg/ml de substrat 17OH progestérone, la souche est mise à croître dans un milieu minimum complémenté avec des acides aminés nécessaires et de l'uracil. Cette souche est capable de convertir la 17OH progestérone en 11-déoxycortisol avec une efficacité de l'ordre de 47% en 24 heures avec une faible production de 4 prégnène 17α-20α-diol-3one dans ces conditions (< 0.5%). Dans certaines conditions (milieu riche défini de type Kappeli avec du galactose comme source de carbone et démarrage de la culture à haute densité : DO 600nm=5), la capacité de réduction de la cétone est augmentée pour atteindre 11% du substrat de départ avec une capacité de bioconversion réduite à 1,5% du substrat de départ. Dans ces conditions, la présence probable du gène GCY1 affecte négativement la bioconversion de la 170H progestérone. Nous avons donc décidé de disrupter le gène GCY1 pour empêcher son activité.

Pour ce faire, la souche TGY212#1 a été transformée par 3µg du plasmide pTG12010#36 linéarisé par les enzymes de restriction SphI et EcoRI. Vingt sept transformants ont été selectionnés sur un milieu minimum complémenté pour les auxotrophies de TGY212#1 mais ne contenant pas d'uracil. Ces colonies ont été soumises à des tests de bioconversion dans un milieu minimum complémenté avec du galactose comme source de carbone car celui-ci est un inducteur connu de GCY1. Tous les clones TGY243 présentaient une capacité de convertir la 17OH progestérone en 11-deoxycortisol sans pour autant produire des quantités détectables de 4 prégnène 17α, 20α-diol-3one. Un clone TGY243#1 a été sélectionné pour introduire à la place du gène URA3 une cassette d'expression pour la P450c21 humaine.

Cette souche TGY243#1 est transformée par le plasmide YRP7 (2µg) et par le plasmide pTG12086 linéarisé par l'enzyme XhoI (5µg). Le fragment transformateur de pTG12086 contient la séquence codante de GCY1 interrompue par une cassette d'expression pour la P450c21 humaine (TDH3 : : cDNAP450c21 humaine : : terminateur PGK°). Les colonies qui croissent en absence de tryptophane sont sélectionnées. Ces 381 colonies sont ensuite transférées sur un milieu contenant du tryptophane et du 5 fluoro orotate. Une dizaine de colonies sont ensuite testées dans un milieu riche de type YPG complémenté avec du tryptophane, de l'histidine, de la leucine et de l'uracil à une concentration de 50µg/ml. Les souches sont mises à convertir de la 17OH progestérone à une concentration de 100µg/ml à partir d'une DO600nm de 0.1 pour 16 heures.

Parmi ces 10 clones, un clone TGY245#1D est choisi suivant deux critères, notamment la capacité à convertir le 170H progestérone en 11-deoxycortisol et deuxièmement l'absence de la formation de 4 prégnène 17α-20α-diol-3one indiquant la disruption de GCY1.

### Propriété des souches GCY1 déficientes :

Les résultats obtenus montrent que le « Knock Out » de CGY1 élimine l'activité 20α-HSD inductible par le galactose. Ainsi, la production in vivo de 4-prégnène-17α-20α-diol-3-one à partir de 17α-hydroxyprogestérone (100 µg/ml) a été testée en culture de souches sauvages et de souches TGY170 (gcyl-Δ::LEU2), cultivées soit en milieu glucose ou soit en milieu galactose (Fig. 3). Dans le cas d'une culture en milieu galactose, on a observé une réduction d'environ 95 % de la production de 4-prégnène-17α-20α-diol-3-one de la souche mutante par rapport à la souche sauvage. En milieu glucose, la réduction est plus faible, ce qui semble indiquer que le produit du gène GCY1 comporte une activité 20α-HSD inductible par le galactose. Quelle que soit la source de carbone utilisée, une activité résiduelle est présente dans le mutant gcyl1-Δ, qui est sensiblement identique.

### Propriétés du double mutant GCY1,YPR1 déficient :

Compte tenu du fait que Gcylp et Yprlp ont été trouvées associées à l'activité 20α-HSD (voir ci-avant), et que Yprlp est l'homologue le plus proche de Gcylp (65 % d'identité des acides aminés), une double disruption de GCY1 et de YPR1 a été réalisée et testée pour son activité de type 20α-HSD. Les résultats obtenus montrent que la souche déficiente pour les deux gènes (TGY197) est essentiellement dépourvue d'activité 20α-HSD.

Plus particulièrement, les levures TGY197 (gcy1::LEU2, ypr1::URA3) ont été générées et testées in vivo pour leur activité 20α-HSD. Les cellules ont été cultivées avec soit du glucose soit du galactose comme source de carbone, en présence de 100 µg/ml de 17α-hydroxyprogestérone. Après 72 h, la 4-prégnène-17α-20α-diol-3-one est indétectable dans le liquide de fermentation, démontrant que l'inactivation des deux gènes conduit à une suppression de l'activité 20α-HSD détectable.

### Spécificité de substrat des souches sauvages et mutantes

Une série de composants déjà décrits comme substrats pour différentes classes de réductases (aldose-, aldéhyde- et carbonyl-réductases) a été testée sur des homogénats sauvages et sur des souches mutantes dans différentes conditions de culture (Tableau II). On a observé que Gcylp et Ypr1P sont les seules réductases de levures aldo-kéto qui acceptent la 17α-hydroxyprogestérone comme substrat.

Gcylp semble utiliser tous les substrats testés, puisque dans tous les cas, l'induction par galactose accroît l'activité. En milieu glucose, on a observé une activité basale pour tous les composants à l'exception de la 17α-hydroxyprogestérone, indépendamment de la présence de GcylP et de Yprlp. En milieu galactose, on a observé une activité plus importante dans les souches mutantes pour les deux aldéhydes testées, cependant moins prononcée que dans les souches sauvages. Ceci indique qu'une enzyme spécifique pour les aldéhydes autres que GcylP est inductible par le galactose.

Dans un rapport récent sur la physiologie des levures sous stress osmotique, GCY1 a été identifié comme étant réactif. Le séquençage d'un peptide isolé à partir d'une glycérol déhydrogénase d'Aspergillus niger a montré une homologie avec deux protéines de levures : Gcylp et Yprlp. L'induction de GCY1 sous stress osmotique (en plus de son induction par galactose) pourrait indiquer que GCY1 comporte une activité glycérol déhydrogénase, ainsi que suggéré dans Norbeck et Blomberg. Toutefois, une telle activité n'a pas été mise en évidence à ce jour.

La réduction de 17α-hydroxyprogestérone en 4-prégnène-17α-20α-diol-3-one dans S. Cerevisiae est due principalement au produit du gène GCY et, dans une moindre mesure, au produit du gène YPR1. Selon la nomenclature proposée par Jez et al. (1997), ces enzymes devraient être classées dans la sous-famille AKR1C. Chez les mammifères, on a proposé des HSD, appartenant à la famille des AKR, pour réguler la disponibilité des hormones stéroïdiennes. Le rôle physiologique de ces enzymes dans la levure demeure inconnu, puisque les levures ne sont pas supposées pouvoir être mises en présence de stéroïdes dans un environnement naturel. Quelle que soit la signification biologique d'une telle activité dans la levure, son élimination contribue à l'amélioration de la production de corticostéroïdes à partir de levures, notamment de levures génétiquement modifiées selon l'invention.

### Exemple de bioconversion avec la P450c21 humaine dans la levure en présence et en absence de GCY1 et YPR1.

Dans le but de montrer que la disruption de GCY1 et YPR1 est indispensable pour obtenir une bioconversion spécifique dans la levure *S. cerevisiae,* nous avons comparé la capacité de bioconversion du 17OH-progestérone des souches Fy1679-28c/pTG10497 (Fy/pTG10497), TGY212 #1 et TGY245#2D.
La souche Fy/pTG10497 porte les deux gènes sauvages GCY 1 et YPR1 et le plasmide monocopie de type ARSCEN (« Autonomously Replicating Sequence Centromére ») d'expression de la P450c21 humaine. Le cDNA de la P450c21 humaine est sous contrôle du promoteur TEF1 dans ce plasmide.
La souche TG212#1 porte une copie de la cassette d'expression du gène P450c21 humaine (TEF1: : P450c21 humaine : : terminateur PGK) intégrée au locus YPR1 et possède une copie sauvage du gène GCY1.
TGY245#2D ne possède pas de copies des gènes YPR1 et GCY1: à la place, une copie de la cassette TEF1 : :P450c21 humaine et une copie de TDH3 : P450c21 sont intégrées à chacun des loci, respectivement.
Ces souches ont été cultivées dans un milieu minimum avec un supplément de casamino acides pendant 48 heures. Les souches sont resuspendues dans du milieu frais Kappeli avec un supplément d'uracile, d'histidine et de tryptophane en présence de 200mg/l de 170H progestérone. Après une incubation de 72 heures, le milieu en présence des cellules de levure est extrait et analysé comme précédemment par chromatographie liquide haute pression en phase inverse. La présence de 170H progestérone, 11-deoxycortisol et de 4 prégnène 17α-20α-diol-3one est mesurée.

Les résultats sont présentés dans le tableau III. Chaque produit est exprimé en pourcentage de la somme de l'ensemble des produits.

D'après cette expérience, il apparaît clairement que la disruption de GCY1 et YPR1 diminue de façon significative la quantité de 4 prégnène 17α-20α-diol-3one de 7-11% à un niveau non-détectable par nos techniques (sensibilité de 0,5 à 1 mg/l).

### REFERENCES

Amberg et al. (1995), Yeast 11, 1275-1280.
Cauet et al (1999), Eur.J. Biochem. 261, 317-324.
Degryse. et al. (1995), J.Biotech. 39, 181-187.
Degryse, E. (1996), Gene 170, 45-50.
Degryse. et al. (1995), Yeast 11, 629-640.
Dumas et al (1994), Cytochrome P450, 8th International Conference, Ed. M. C. Lechner, John Libbey Eurotext, Paris, pp. 527-530.
Dumas et al (1996), Eur.J.Biochem. 238, 495-504.
Duport et al (1998), Nat.Biotech. 16, 1-6.
Hanahan, D. (1983) J.Mol.Biol. 166, 557-580.
Hubacek et al (1970), J.Mol.Biol. 50, 111-127.
Ito et al (1983), J.Bact. 153, 163-168.
Miller et al (1988), Endocrine Revs 9, 295-318.
Sakaki et al (1989), DNA 8, 409-418.
Sakaki et al (1991), Pharmacogen. 1, 86-93.
Sambrook et al (1989), Cold Spring Harbor University Press, 2nd edition, Cold Spring Harbor.
Sherman, F. (1991), Methods Enzymol. 194, 3-21.
Thierry et al (1995), Yeast 11, 121-135.
WU et al (1991), Saccharomyces cerevisiae DNA Cell Biol. 10, 201-209.
Database WPI Section Ch, Week 199011 Document Publications Ltd., London, GB, AN 1990-079090 (JP 02 031680 A).
Dechsner et al. (1988), FEBS LETTERS 238, 123-128.
WO99/25865 A (1999) Microbia, Inc.

**Tableau I**

| ORF | Nom du Gène | % d'identité d'acides aminés avec la 20αHSD |
|---|---|---|
| YOR120W | GCY1 | 44 |
| YDR368W | YPR1 | 43 |
| YHR104W | - | 41 |
| YBR149W | - | 40 |
| YJR096W | - | 39 |
| YDL124W | - | 32 |

**Tableau III**

| Produit | Fy/10497 | TGY212#1 | TGY245#2D |
|---|---|---|---|
| 17OH Progesterone | 74 | 87 | 28 |
| 11-deoxycortisol | 19 | 20 | 82 |
| 4 prégnène 17α-20α-diol-3one | 7 | 11 | 0 |

**Tableau II**

| | Fy1679.28c | | TGY170 | | TGY197 | |
|---|---|---|---|---|---|---|
| | glucose | galactose | glucose | galactose | glucose | galactose |
| xylose | 8 | 50 | 5 | 8 | 3 | 5 |
| méthylglyoxal | 75 | 376 | 92 | 88 | 56 | 104 |
| glycéraldéhyde | 32 | 1119 | 40 | 357 | 42 | 384 |
| nitrobenzaldéhyde | 59 | 1117 | 69 | 334 | 50 | 395 |
| 17α-hydroxyprogesté rone | 0,04 | 1,44 | 0,006 | 0,01 | nd | nd |

| | | | | | | |
|---|---|---|---|---|---|---|
| Les activités sont données en µM/min/mg protéine nd: non détecté | | | | | | |

## Revendications

1. Souche de levure présentant une activité de type 20α hydroxystéroïde déhydrogénase (20α HSD) réduite **caractérisée en ce qu'**elle possède :
- une modification génétique inactivatrice du gène GCY₁, ou
- une modification génétique inactivatrice du gène YPR1, ou
- une modification génétique inactivatrice des gènes GCY1 et YPR1,
à l'exclusion des souches gcyD0 et gcyDD0.

2. Souche de levure selon la revendication 1, **caractérisée en ce que** la modification génique comprend au moins une mutation, substitution, délétion et/ou insertion d'une ou plusieurs paires de bases dans la région régulatrice ou codante du ou des gènes considérés.

3. Souche de levure selon la revendication 2, **caractérisée en ce que** la modification génétique est une délétion de tout ou partie du ou des gènes considérés.

4. Souche de levure selon la revendication 3, **caractérisée en ce que** la délétion de tout ou partie du ou des gènes considérés est remplacée par des séquences étrangères, selon la technique de disruption génique.

5. Souche de levure selon la revendication 4, **caractérisée en ce que** la séquence étrangère est un gène de sélection auxotrophe complémentant une exigence nutritionnelle de la souche de levure hôte ; un gène de sélection dominant, tel qu'un gène de résistance à un antibiotique ou un gène rapporteur.

6. Souche de levure selon la revendication 5, **caractérisée** le gène de sélection auxotrophe est le gène URA3, le gène LEU2, le gène TRP1, le gène HIS3 ou le gène ADE2.

7. Souche de levure selon l'une des revendications 1 à 6, **caractérisée en ce qu'**il s'agit d'une levure du genre Saccharomyces.

8. Souche de levure selon la revendication 7, **caractérisée en ce qu'**il s'agit d'une levure Saccharomyces cerevisiae.

9. Souche de levure selon l'une des revendications 1 à 8, comprenant un gène GCY1 et un gène YPR1 disruptés.

10. Procédé de production de dérivés stéroïdiens à partir de composés hydroxy-stéroïde, utilisant une levure présentant une activité de type 20α hydroxystéroïde dehydrogénase (20αHSD) réduite et présentant un gène GCY1 et/ou YPR1 non fonctionnel, ou une préparation dérivée d'une telle levure.

11. Procédé selon la revendication 10 **caractérisé en ce que** la levure utilisée possède :
- une modification génétique inactivatrice du gène GCY1, ou
- une modification génétique inactivatrice du gène YPR1, ou
- une modification génétique inactivatrice des gènes GCY1 et YPR1.

12. Procédé selon la revendication 10 ou 11, dans lequel l'hydroxy-stéroïde est l'hydroxyprogestérone ou ses précurseurs.

13. Procédé selon la revendication 12 dans lequel l'hydroxystéroïde est le 17α-hydroxyprogestérone et le dérivé stéroidien est le 11-déoxycortisol.

14. Procédé selon l'une des revendications 10 à 13,
**caractérisé en ce que** la levure présente un gène GCY1 et un gène YPR1 disruptés.

15. Procédé selon l'une des revendications 10 à 14,
**caractérisé en ce que** la levure est une levure du genre Saccharomyces.

16. Utilisation d'une levure présentant une activité de type 20α hydroxystéroïde dehydrogénase (20αHSD) réduite et présentant un gène GCY1 et/ou YPR1 non fonctionnel, ou une préparation dérivée d'une telle levure, pour la préparation, la production, la synthèse et/ou la modification de composés stéroïdiens in vitro ou ex vivo.

17. Utilisation selon la revendication 16, **caractérisé en ce que** la levure présente :
- une modification génétique inactivatrice du gène GCY1, ou
- une modification génétique inactivatrice du gène YPR1, ou
- une modification génétique inactivatrice des gènes GCY1 et YPR1.

18. Utilisation selon la revendication 17, **caractérisé en ce que** la levure présente un gène GCY1 et un gène YPR1 disruptés.

19. Utilisation selon la revendication 18 **caractérisé en ce que** la levure est une levure du genre Saccharomyces.

## Claims

1. Yeast strain having a reduced 20α-hydroxysteroid dehydrogenase (20α-HSD) activity, **characterized in that** it possesses:
- an inactivating genetic modification of the GCY1 gene,
or
- an inactivating genetic modification of the YPR1 gene,
or
- an inactivating genetic modification of the GCY1 and YPR1 genes,
with the exclusion of the gcyD0 and gcyDD0 strains.

2. Yeast strain according to Claim 1, **characterized in that** the gene modification comprises at least one mutation, substitution, deletion and/or insertion of one or more base pairs in the regulatory or coding region of the gene(s) under consideration.

3. Yeast strain according to Claim 2, **characterized in that** the genetic modification is a deletion of all or part of the gene(s) under consideration.

4. Yeast strain according to Claim 3, **characterized in that** the deletion of all or part of the gene(s) under consideration is replaced with foreign sequences, according to the gene disruption technique.

5. Yeast strain according to Claim 4, **characterized in that** the foreign sequence is an auxotrophic selection gene complementing a nutritional requirement in the host yeast strain; a dominant selection gene, such as a gene for resistance to an antibiotic; or a reporter gene.

6. Yeast strain according to Claim 5, **characterized in that** the auxotrophic selection gene is the URA3 gene, the LEU2 gene, the TRP1 gene, the HIS3 gene or the ADE2 gene.

7. Yeast strain according to one of Claims 1 to 6, **characterized in that** it is a yeast of the Saccharomyces genus.

8. Yeast strain according to Claim 7, **characterized in that** it is a Saccharomyces cerevisiae yeast.

9. Yeast strain according to one of Claims 1 to 8, comprising a disrupted GCY1 gene and a disrupted YPR1 gene.

10. Method for producing steroid derivatives from hydroxysteroid compounds, using a yeast having a reduced 20α-hydroxysteroid dehydrogenase (20αHSD) activity and having a nonfunctional GCY1 and/or YPR1 gene, or a preparation derived from such a yeast.

11. Method according to Claim 10, **characterized in that** the yeast used possesses:
- an inactivating genetic modification of the GCY1 gene,
or
- an inactivating genetic modification of the YPR1 gene,
or
- an inactivating genetic modification of the GCY1 and YPR1 genes.

12. Method according to Claim 10 or 11, in which the hydroxysteroid is hydroxyprogesterone or precursors thereof.

13. Method according to Claim 12 in which the hydroxysteroid is 17α-hydroxyprogesterone and the steroid derivative is 11-deoxycortisol.

14. Method according to one of Claims 10 to 13, **characterized in that** the yeast has a disrupted GCY1 gene and a disrupted YPR1 gene.

15. Method according to one of Claims 10 to 14, **characterized in that** the yeast is a yeast of the Saccharomyces genus.

16. Use of a yeast having a reduced 20α-hydroxysteroid dehydrogenase (20αHSD) activity and having a nonfunctional GCY1 and/or YPR1 gene, or a preparation derived from such a yeast, for the preparation, production, synthesis and/or modification of steroid compounds in vitro or ex vivo.

17. Use according to Claim 16, **characterized in that** the yeast has:
- an inactivating genetic modification of the GCY1 gene,
or
- an inactivating genetic modification of the YPR1 gene, or
- an inactivating genetic modification of the GCY1 and YPR1 genes.

18. Use according to Claim 17, **characterized in that** the yeast has a disrupted GCY1 gene and a disrupted YPR1 gene.

19. Use according to Claim 18, **characterized in that** the yeast is a yeast of the Saccharomyces genus.

## Patentansprüche

1. Hefestamm mit einer verringerten 20α-Hydroxysteroiddehydrogenase (20α HSD)-Aktivität, **dadurch gekennzeichnet, daß** er folgendes aufweist:
- eine genetische Modifikation, die das GCY1-Gen inaktiviert,
oder
- eine genetische Modifikation, die das YPR1-Gen inaktiviert,
oder
- eine genetische Modifikation, die das GCY1- und das YPR1-Gen inaktiviert,
mit Ausnahme der Stämme gcyD0 und gcyDD0.

2. Hefestamm nach Anspruch 1, **dadurch gekennzeichnet, daß** die Genmodifikation mindestens eine Mutation, Substitution, Deletion und/oder Insertion von einem oder mehreren Basenpaaren in der Regulator- oder Codierregion des bzw. der betreffenden Gene umfaßt.

3. Hefestamm nach Anspruch 2, **dadurch gekennzeichnet, daß** es sich bei der genetischen Modifikation um eine vollständige oder teilweise Deletion des bzw. der betroffenen Gene handelt.

4. Hefestamm nach Anspruch 3, **dadurch gekennzeichnet, daß** die teilweise oder vollständige Deletion des bzw. der betroffenen Gene mittels der Gendisruptionstechnik durch heterologe Sequenzen ersetzt wird.

5. Hefestamm nach Anspruch 4, **dadurch gekennzeichnet, daß** es sich bei der heterologen Sequenz um ein Auxotrophieselektionsgen, das einen Nährstoffanspruch des Wirtshefenstamms komplementiert, ein dominantes Selektionsgen wie ein Antibiotikumresistenzgen oder ein Reportergen handelt.

6. Hefestamm nach Anspruch 5, **dadurch gekennzeichnet, daß** es sich bei dem Auxotrophieselektionsgen um das URA3-Gen, das LEU2-Gen, das TRP1-Gen, das HIS3-Gen oder das ADE2-Gen handelt.

7. Hefestamm nach einem der Ansprüche 1 bis 6, **dadurch gekennzeichnet, daß** es sich um eine Hefe der Gattung Saccharomyces handelt.

8. Hefestamm nach Anspruch 7, **dadurch gekennzeichnet, daß** es sich um eine Saccharomyces cerevisiae-Hefe handelt.

9. Hefestamm nach einem der Ansprüche 1 bis 8, der ein disrumpiertes GCY1- und YPR1-Gen umfaßt.

10. Verfahren zur Herstellung von Steroidderivaten aus Hydroxysteroidverbindungen, bei denen man eine Hefe einsetzt, die eine verringerte 20α-Hydroxysteroiddehydrogenase (20αHSD)-Aktivität aufweist und ein nicht funktionelles GCY1- und/oder YPR1-Gen aufweist, oder ein von solch einer Hefe stammendes Präparat.

11. Verfahren nach Anspruch 10, **dadurch gekennzeichnet, daß** die verwendete Hefe folgendes aufweist:
- eine genetische Modifikation, die das GCY1-Gen inaktiviert,
oder
- eine genetische Modifikation, die das YPR1-Gen inaktiviert,
oder
- eine genetische Modifikation, die das GCY1- und das YPR1-Gen inaktiviert.

12. Verfahren nach Anspruch 10 oder 11, wobei es sich bei dem Hydroxysteroid um Hydroxyprogesteron oder seine Vorstufen handelt.

13. Verfahren nach Anspruch 12, wobei es sich bei dem Hydroxysteroid um 17α-Hydroxyprogesteron und bei dem Steroidderivat und um 11-Desoxycortisol handelt.

14. Verfahren nach einem der Ansprüche 10 bis 13, **dadurch gekennzeichnet, daß** die Hefe ein disrumpiertes GCY1- und YPR1-Gen aufweist.

15. Verfahren nach einem der Ansprüche 10 bis 14, **dadurch gekennzeichnet, daß** es sich bei der Hefe um eine Hefe der Gattung Saccharomyces handelt.

16. Verwendung einer Hefe, die eine verringerte 20α-Hydroxysteroiddehydrogenase (20αHSD)-Aktivität aufweist und ein nicht funktionelles GCY1- und/oder YPR1-Gen aufweist, oder eines von solch einer Hefe stammenden Präparats für die in-vitro oder ex-vivo Herstellung, Produktion, -Synthese und/oder -Modifikation von Steroidverbindungen.

17. Verwendung nach Anspruch 16, **dadurch gekennzeichnet, daß** die Hefe folgendes aufweist:
- eine genetische Modifikation, die das GCY1-Gen inaktiviert,
oder
- eine genetische Modifikation, die das YPR1-Gen inaktiviert,
oder
- eine genetische Modifikation, die das GCY1- und das YPR1-Gen inaktiviert.

18. Verwendung nach Anspruch 17, **dadurch gekennzeichnet, daß** die Hefe ein disrumpiertes GCY1- und YPR1-Gen aufweist.

19. Verwendung nach Anspruch 18, **dadurch gekennzeichnet, daß** es sich bei der Hefe um eine Hefe der Gattung Saccharomyces handelt.


### LISTE DE SEQUENCES

<110> AVENTIS PHARMA SA
<120> LEVURES MODIFIEES ET UTILISATIONS, NOTAMMENT POUR LA PRODUCTION DE DERIVES STEROIDIENS
<130> 17142WO
<140>
   <141>
<150> 00FR0010437
   <151> 2000-08-08
<160> 23
<170> PatentIn Ver. 2.1
<210> 1
   <211> 50
   <212> ADN
   <213> Séquence artificielle
<220>
   <223> Description de la séquence artificielle: oligonucleotide
<400> 1
   gattcggtaa tctccgaaca ggtaccaatt atatcagtta ttacccggga 50
<210> 2
   <211> 20
   <212> ADN
   <213> Séquence artificielle
<220>
   <223> Description de la séquence artificielle: oligonucleotide
<400> 2
   agccatcttt caaagcggtt 20
<210> 3
   <211> 22
   <212> ADN
   <213> Séquence artificielle
<220>
   <223> Description de la séquence artificielle: oligonucleotide
<400> 3
   ccgatcgaat caaaacgaac ag 22
<210> 4
   <211> 20
   <212> ADN
   <213> Séquence artificielle
<220>
   <223> Description de la séquence artificielle: oligonucleotide
<400> 4
   tctaatcagc tagtaagaac 20
<210> 5
   <211> 67
   <212> ADN
   <213> Séquence artificielle
<220>
   <223> Description de la séquence artificielle: oligonucleotide
<400> 5
   aaccgctttg aaagatggct atcgattttc aattcaattc atcatttttt ttttattctt 60
   ttttttg 67
<210> 6
   <211> 60
   <212> ADN
   <213> Séquence artificielle
<220>
   <223> Description de la séquence artificielle: oligonucleotide
<400> 6
   ctgttcgttt tgattcgatc gggaagcttg ggtaataact gatataatta aattgaactc 60
<210> 7
   <211> 32
   <212> ADN
   <213> Séquence artificielle
<220>
   <223> Description de la séquence artificielle: oligonucleotide
<400> 7
   tacgctcgag acgttggtgt cattgatatt ca 32
<210> 8
   <211> 21
   <212> ADN
   <213> Séquence artificielle
<220>
   <223> Description de la séquence artificielle: oligonucleotide
<400> 8
   cttcattcaa atagatagcc g 21
<210> 9
   <211> 22
   <212> ADN
   <213> Séquence artificielle
<220>
   <223> Description de la séquence artificielle: oligonucleotide
<400> 9
   tatggctaaa aagcacggcg tt 22
<210> 10
   <211> 31
   <212> ADN
   <213> Séquence artificielle
<220>
   <223> Description de la séquence artificielle: oligonucleotide
<400> 10
   cgatctcgag tttctcgttg ttcaggtact g 31
<210> 11
   <211> 68
   <212> ADN
   <213> Séquence artificielle
<220>
   <223> Description de la séquence artificielle: oligonucleotide
<400> 11
   cggctatcta tttgaatgaa gatcgatttt caattcaatt catcattttt tttttattct 60
   tttttttg 68
<210> 12
   <211> 58
   <212> ADN
   <213> Séquence artificielle
<220>
   <223> Description de la séquence artificielle: oligonucleotide
<400> 12
   aacgccgtgc tttttagcca taagcttggg taataactga tataattaaa ttgaactc 58
<210> 13
   <211> 23
   <212> ADN
   <213> Séquence artificielle
<220>
   <223> Description de la séquence artificielle: oligonucleotide
<400> 13
   tttgctcgag gttacagaag ggc 23
<210> 14
   <211> 24
   <212> ADN
   <213> Séquence artificielle
<220>
   <223> Description de la séquence artificielle: oligonucleotide
<400> 14
   gattctcgag caattggctg acta 24
<210> 15
   <211> 40
   <212> ADN
   <213> Séquence artificielle
<220>
   <223> Description de la séquence artificielle: oligonucleotide
<400> 15
   ggaattccgt cgacaaaaat gctgctcctg ggcctgctgc 40
<210> 16
   <211> 29
   <212> ADN
   <213> Séquence artificielle
<220>
   <223> Description de la séquence artificielle: oligonucleotide
<400> 16
   cctcaatggt cctcttggag ttcagcacc 29
<210> 17
   <211> 32
   <212> ADN
   <213> Séquence artificielle
<220>
   <223> Description de la séquence artificielle: oligonucleotide
<400> 17
   gtcgacaaaa atgctgctcc tgggcctgct gc 32
<210> 18
   <211> 14
   <212> ADN
   <213> Séquence artificielle
<220>
   <223> Description de la séquence artificielle: oligonucleotide
<400> 18
   aaatcgataa catg 14
<210> 19
   <211> 14
   <212> ADN
   <213> Séquence artificielle
<220>
   <223> Description de la séquence artificielle: oligonucleotide
<400> 19
   ttatcgattt catg 14
<210> 20
   <211> 30
   <212> ADN
   <213> Séquence artificielle
<220>
   <223> Description de la séquence artificielle: oligonucleotide
<400> 20
   attgatatcg ataaaaagca cggcgttgag 30
<210> 21
   <211> 26
   <212> ADN
   <213> Séquence artificielle
<220>
   <223> Description de la séquence artificielle: oligonucleotide
<400> 21
   tctcggaatt caggtactgc agccag 26
<210> 22
   <211> 32
   <212> ADN
   <213> Séquence artificielle
<220>
   <223> Description de la séquence artificielle: oligonucleotide
<400> 22
   tacgctcgag acgttggtgt cattgatatt ca 32
<210> 23
   <211> 30
   <212> ADN
   <213> Séquence artificielle
<220>
   <223> Description de la séquence artificielle: oligonucleotide
<400> 23
   caactaagct tcattcaaat agatagccgc 30